# EUROPEAN PATENT APPLICATION

(11) **EP 1 595 535 A2**
(43) Date of publication of application: **16.11.2005**
(21) Application number: 03811268.6
(22) Date of filing: 19.12.2003
(51) Int. Cl.: A61K 9/24, A61K 31/135, A61K 31/13

(54) **DELIVERY DEVICE CONTAINING VENLAFAXINE AND MEMANTINE AND USE METHOD THEREOF**

(30) Priority: 23.12.2002 US 436156 P
(71) Applicant: Osmotica Costa Rica Sociedad Anonima, San Josè (CR)
(72) Inventor: VERGEZ, Juan A., 1643 Buenos Aires (AR); FAOUR, Joaquina, 1426 Buenos Aires (AR); RICCI, Marcelo, A., 1419 Buenos Aires (AR); PASTINI, Ana, C., 1416 Buenos Aires (AR)
(74) Representative: Weber, Thomas
(86) International application number: PCT/CR2003/000004
(87) International publication number: WO 2004/056335

(57) **Abstract**

The present invention provides an osmotic device containing controlled release venlafaxine in the core in combination with an anti-Alzheimer's or an anti-Parkinson's drug in a rapid release external coat. Memantine is used as an anti-Alzheimer's drug or an anti-Parkinson's drug. Particular embodiments of the invention provide osmotic devices having predetermined release profiles. One embodiment of the osmotic device includes an external coat that has been spray-coated rather than compression-coated onto the device. The device is useful for the treatment of depression in Alzheimer's and/or Parkinson's patients. The device and method can also be used to treat or ameliorate other symptoms associated with Alzheimer's disease, Parkinson's disease or any other neurological disorder. Other dosage forms that provide a controlled, sustained or extended release of venlafaxine in combination with a rapid or immediate release of memantine are useful in the invention.

## Description

### FIELD OF THE INVENTION

This invention pertains to a drug delivery device containing an anti-depressant and an anti-Alzheimer's agent. More particularly, it pertains to a drug delivery device for the controlled delivery of venlafaxine and the rapid delivery of a drug used to treat Alzheimer's disease or Parkinson's disease.

### BACKGROUND OF THE INVENTION

Dementia is a term used to describe a group of symptoms common to certain diseases or conditions. Dementia is an acquired syndrome in which intellectual ability decreases to the point that it interferes with daily function. Symptoms include loss of memory, judgment, and reasoning, difficulty with day-to-day function and changes in mood and behavior. These symptoms may affect functioning at work, in social situations or in day-to-day activities.

Alzheimer's disease is the leading cause of dementia. It is a progressive and degenerative brain disorder that affects a person's mental and physical abilities and behavior by destroying vital brain cells. This damage interferes with brain cell functioning and the passage of chemical impulses between brain cells. These changes occur mainly in parts of the brain that control memory, learning, emotional expression and behavior.

There are two known types of Alzheimer's Disease including familial Alzheimer's Disease or early-onset Alzheimer's Disease, and the late-onset Alzheimer's Disease. Familial Alzheimer's Disease is clearly traced over several generations of a family but is rare and only accounts for 5 to 10 per cent of all cases. The late-onset Alzheimer's Disease is more common.

Some of the symptoms of Alzheimer's disease include memory loss that affects day-to-day function; difficulty performing familiar tasks; problems with language; disorientation of time and place; poor or decreased judgment; problems with abstract thinking; misplacing things; changes in mood and behavior; changes in personality; and loss of initiative.

Treatments for Alzheimer's disease include pharmacological and nonpharmacological methods. A nonpharmacological approach is generally preferred. However, if nonpharmacological therapy fails, pharmacological therapy is introduced. Pharmacological therapy can also be used if there is a risk of danger or if the patient is very distressed. Cholinesterase inhibitors, such as tacrine and donepezil, and other agents, such as estrogen, nonsteroidal anti-inflammatory drugs and botanical agents, such as ginkgo biloba have been used.

Memantine has been approved for the treatment of Alzheimer's Disease by the regulatory authorities in the European Union. Ebixa® (H. Lundeck A/S) and Axura® (Merz Pharmaceuticals GmbH) brand memantine are the first of a new class of medicines (NMDA receptor antagonists) for the treatment of Alzheimer's disease, Ebixa® memantine reportedly elicits a clinically significant effect in patients with moderately severe and severe Alzheimer's disease. Memantine is used in Germany to treat Parkinson's disease, dementia in the elderly, and to speed the recovery of comatose patients. Memantine may also be useful for use in patients with stroke, traumatic brain injury, neurogenic pain, peripheral neuropathies, and neurodegenerative conditions such as AIDS-related dementia, and other dementias. Akatinol™ brand memantine (Merz Pharmaceuticals, Germany) is marketed in boxes of 50 x 10 mg tablets (which can be split in half for patients who wish to start at 5 mg a day). The Food and Drug Administration recently approved memantine for treatment of moderate to severe Alzheimer's Disease; memantine will be marketed under the trade name Namenda by Forest Labs.

Depression is very common among people with Alzheimer's disease. About half of these people have serious depression. In many cases, they become depressed when they realize that their memory and ability to function are declining. Depression may make it even harder for a person with Alzheimer's disease to function, to remember things and to enjoy life. Even though not considered general antidepressant agents, galanthamine and memantine have been shown to possess some antidepressant properties.

Clinical depression is a disorder characterized by low self-esteem, guilt, self-reproach, introversion, sadness, despair, sleeping disorders, eating disorders or discouragement. Depression generally causes a lower or decrease of a person's function.

Antidepressant medicines have proven to be helpful in treating depression in patients with Alzheimer's disease. These medicines can improve the symptoms of sadness and depression, and may also improve appetite and sleep problems.

Antidepressants, such as venlafaxine, have been tested for the treatment of depression. Venlafaxine is commercially available in an extended release capsule dosage form from Wyeth Ayerst under the trademark EFFEXOR XR™. Venlafaxine HCl extended-release is indicated for depression and Generalized Anxiety Disorder as defined in DSM-IV. The capsule is available in 37.5, 75, and 150 mg strengths. The capsule is disclosed in U.S. Patent No. 4,535,186 and does not contain the venlafaxine in combination with an anti-Alzheimer's drug.

U.S. Patent No. 6,274,171 to Sherman et al. covers various methods of use/administration of venlafaxine in an encapsulated dosage form.

Conventional antidepressant therapy has been indicated for the treatment of depression in subjects suffering from Alzheimer's disease. Antidepressants such as mirtazapine (Raji et al. in *Ann. Pharmacother.* (2001) Sep; 35(9):1024-7), fluoxetine (Petracca et al. in *Int. Psychogeriatr.* (2001) Jun; 13(2):233-40), sertraline (Lyketsos et al. in *Am. J. Psychiatry.* (2000) Oct;157(10):1686-9); mianserine (Haupt in *J. Am. Geriatr. Soc.* (1991) Nov; 39(11):1141), citalopram (Nyth et al. in *Br. J. Psychiatry* (1990) Dec;157:894-901), SSRI's, and MAO inhibitors have been used alone. Efficacy of antidepressants in Alzheimer's associated depression has not been completely predictable. For example, mirtazapine, mianserine, and sertraline reportedly provided a statistically significant therapeutic benefit in limited trials, whereas, fluoxetine provided no significant benefit and citalopram provided mixed results.

Venlafaxine has not been evaluated in the treatment of depression in Alzheimer's patients. Rog6z et al *(Eur. Neuropsychopharmacol.* 11, Suppl. 2, S47, P.1.23, 2001) reported that memantine in combination with venlafaxine demonstrated a synergistic effect in the forced swimming test in male Wistar rats, which is an animal model for depression. The combination of memantine with venlafaxine was administered to the male rats three times, 1, 5 and 24 hours before the test. A more potent antidepressant-like effect was induced by the administration of the combination of drugs than by the administration of the drugs alone. A synergistic effect was reportedly observed when memantine or venlafaxine were used in a dose that was ineffective when either of the drugs was given alone. To date, however, no specific combinations have been found to be particularly suitable or useful for treatment of Alzheimer's disease or depression associated therewith.

U.S. Patents No. 6,441,048, No. 6,342,533, and No. 6,197,828 to Jerussi et al. disclose and claim pharmaceutical compositions containing derivatives of (+)-VFX or (-)-VFX and methods of using the same for the treatment of cerebral function disorders such as Parkinson's disease. The Jerussi et al. patent defines the term "method of treating Parkinson's disease" to mean "relief from the symptoms of Parkinson's disease which include, but are not limited to, slowly increasing disability in purposeful movement, tremors, bradykinesia, rigidity, and a disturbance of posture in humans." They also define the term "a method for treating cerebral function disorders" to mean "relief from the disease states associated with cerebral function disorders involving intellectual deficits which include but are not limited to, senile dementia, Alzheimer's type dementia, memory loss, amnesia/amnestic syndrome, disturbances of consciousness, coma, lowering of attention, speech disorders, Parkinson's disease, Lennox syndrome, autism, hyperkinetic syndrome and schizophrenia. Also within the meaning of cerebral function disorders are disorders caused by cerebrovascular diseases including, but not limited to, cerebral infarction, cerebral bleeding, cerebral arteriosclerosis, cerebral venous thrombosis, head injuries, and the like and where symptoms include disturbances of consciousness, senile dementia, coma, lowering of attention, speech disorders, and the like."

U.S. Patent No. 5,530,013 to Husbands et al. discloses and claims the use of venlafaxine for inducing enhancement of cognition, such as in patients suffering from Parkinson's disease. The Husbands et al. patent discloses that "It should also be understood that the present invention is intended to include all methods of, and reasons for, inducing cognition enhancement in a mammal by administering to the mammal an effective amount of venlafaxine or its analogues or pharmaceutically acceptable salts. Husbands et al. also state that, "inducing cognition enhancement is to be understood as covering all prophylactic, therapeutic, progression inhibiting, remedial, maintenance, curative or other administrations, regimens or treatments of or with venlafaxine or its analogues or salts that yield the desired cognition enhancing effects in a mammal."

The use of venlafaxine for the treatment of depression associated with Parkinson's disease has been disclosed (Allain et al. in *British Medical Journal,* (13 May 2000), 320/7245, 1287-1288; Schurer-Maly in *Therapiewoche,* (2001) 17/6 186-189; Poewe et al. in *Journal of Neurology,* Supplement, (2001) 248/3 (12-21); Okun et al. in *Neurology,* (26 Feb 2002) 58/4 SUPPL. 1 (S63-S70); Cunningham in *J. Clin. Psych.,* (1994 Sep), 55 Suppl A, 90-7).

Controlled release capsule dosage forms and osmotic device dosage forms are generally known by the skilled artisan to provide different release profiles. Effective therapy with antidepressants is dependent upon a careful control of the blood plasma levels of these agents, and therefore, upon the release profiles of these agents from their respective dosage forms.

Osmotic devices and other tablet formulations are known for their ability to provide a controlled release of a wide range of drugs. Such osmotic devices and other tablet formulations are disclosed in U.S. Patent No. 4,014,334 to Theeuwes et al., U.S. Patent No. 4,576,604 to Guittard et al., Argentina Patent No. 234,493, U.S. Patent No. 4,673,405 to Guittard et al., U.S. Patent No. 5,558,879 to Chen et al., U.S. Patent No. 4,810,502 to Ayer et al., U.S. Patent No. 4,801,461 to Hamel et al., U.S. Patent No. 5,681,584 to Savastano et al., US Patent No. 3,845,770, U.S. Patent No. 6,004,582 to Faour et al., and Argentina Patent No. 199,301, the entire disclosures of which are hereby incorporated by reference.

U.S. Patent No. 6,110,498 to Rudnic et al. discloses and claims an osmotic device having a semipermeable wall surrounding a core comprising a pharmaceutical agent, at least one non-swelling solubilizing agent that enhances the solubility of the pharmaceutical agent, at least one non-swelling osmotic agent and a non-swelling wicking agent dispersed throughout the core. The composition excludes any "agent that provides a physical force other than by osmotic pressure for delivering the pharmaceutical agent whereby the pharmaceutical agent is delivered through the passageway by osmosis rather than by another force."

U.S. Publication No. US 2001-0048943 A1 and PCT International Publication No. WO 01/51041 A1 to Faour et al. disclose osmotic device formulations for the administration of venlafaxine and an anti-psychotic agent. The venlafaxine is provided in controlled release form in the core and the anti-psychotic agent is provided in rapid release form in an external coat surrounding the core of the osmotic device. Faour et al. do not disclose the use of venlafaxine in treating depression associated with Alzheimer's disease.

These references, however, do not disclose osmotic devices that provide the specific plasma profiles or release profiles for venlafaxine (VFX) and memantine that the present invention provides. Moreover, the prior art does not disclose an osmotic device containing a combination of venlafaxine with memantine, and generally wherein the venlafaxine and memantine are delivered according to specific release profiles that are advantageous over known formulations.

### SUMMARY OF THE INVENTION

The invention provides an improved method of treating Alzheimer's disease comprising administering in combination venlafaxine in controlled, extended or sustained release form and memantine in controlled, extended, sustained or rapid release form. The drugs can be administered by way of a controlled release device, such as an osmotic device. The invention also provides a method of treating depression associated with Alzheimer's disease and/or Parkinson's disease or of ameliorating one or more symptoms associated with Alzheimer's disease and/or Parkinson's disease. The composition and dosage forms of the invention can be used to treat other neurological diseases or disorders such as dementia, vascular dementia, HIV dementia, multiple sclerosis, drug dependence, epilepsy diabetic neuropathy, neuropathic pain and chronic pain.

In one aspect, the present invention provides an osmotic device comprising:
a controlled release core comprising a therapeutically effective or sub-therapeutically effective amount of venlafaxine and at least one osmotic agent or osmopolymer;
a membrane surrounding the core and having one or more passageways there through; and
a rapid release drug-containing coat external to the semipermeable membrane and comprising a therapeutically effective or sub-therapeutically effective amount of memantine;
wherein the external coat provides a rapid release of memantine, and at least 75% of the memantine is released within 1 hour after exposure of the osmotic device to an aqueous solution; and
the dosage form provides a dispensable anti-Alzheimer disease and/or anti-Parkinson disease therapeutic composition for administration of venlafaxine in a rate-controlled metered dose per unit time and memantine in a rapid release form.

Another aspect of the invention provides a method of ameliorating one or more symptoms associated with Alzheimer's disease and/or Parkinson's disease in a subject, the method comprising the steps of:
administering to the subject venlafaxine in controlled, extended, prolonged or sustained release form; and
administering to the subject memantine in immediate or rapid release form.

Specific embodiments of the invention include those wherein: 1) the symptom is depression; 2) the venlafaxine and memantine are provided in the same dosage form; 2) the venlafaxine and memantine are provided in different dosage forms; 3) the memantine is provided in rapid release form; 4) the venlafaxine is provided in controlled release form; 5) the venlafaxine and the memantine are present in a therapeutically effective amount; 6) at least one of the venlafaxine and the memantine is present in a sub-therapeutically effective amount; or 7) both venlafaxine and memantine are present in a sub-therapeutically effective amount and the dosage form provides a synergistic clinical benefit.

When the venlafaxine and the memantine for treating Alzheimer's disease and/or Parkinson's disease are provided in different dosage forms, the invention provides a kit comprising at least one first dosage form comprising venlafaxine and at least one second dosage form comprising memantine.

The venlafaxine, as either its free base or salt form, is administered once or twice daily in doses ranging form about 10 to 150 mg, 25 to 125 mg, 150 to 300 mg, or 10-500 mg.

Other features, advantages and embodiments of the invention will become apparent to those skilled in the art by the following description, accompanying examples.

### BRIEF DESCRIPTION OF THE FIGURES

The following drawings are part of the present specification and are included to further demonstrate certain aspects of the invention. The invention may be better understood by reference to one or more of these drawings in combination with the detailed description of the specific embodiments presented herein.
FIG. 1 depicts an *in vitro* release profile for venlafaxine as it is released in a controlled manner from a dosage form according to Example 1.
FIG. 2 depicts an *in vitro* release profile for memantine as it is released in an immediate or rapid manner from a dosage form according to Example 1.

### DETAILED DESCRIPTION OF THE INVENTION

The invention may be better understood by reference to the following definitions provided herein.

By "pharmaceutically acceptable" is meant those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

By the term "effective amount", it is understood the amount or quantity of active agent which is sufficient to elicit the required or desired therapeutic response, or in other words, the amount which is sufficient to elicit an appreciable biological response when administered to a patient.

By "immediate release" is meant a release of an active agent to an environment over a period of seconds to no more than 15 minutes once release has begun and release begins within a few seconds to no more than 15 minutes after administration.

By "rapid release" is meant a release of an active agent to an environment over a period of 1-59 minutes or 1 minute to three hours once release has begun and release can begin within a few minutes after administration or after expiration of a delay period (lag time) after administration.

By "controlled release" is meant a release of an active agent to an environment over a period of about three hours up to about 12 hours, 16 hours, 18 hours, 20 hours, a day, or more than a day.

By "sustained release" is meant a controlled release of an active agent that maintains a constant drug level in the blood or target tissue.

By "extended release" is meant a controlled release of an active agent from a dosage form to an environment that allow at least a two-fold reduction in frequent dosing compared to the drug presented in a conventional dosage form (e.g., a solution or rapid releasing conventional solid dosage forms).

By "delayed release" is meant a release of an active agent to an environment that exhibits an initial delay (lag time) in the release of drug after administration, in other words, that the release of the active agent starts at any time other than promptly after administration. The period of delay is generally about 5 minutes to 12 hours, or 30 minutes to 10 hours, or 30 minutes to 8 hours or 30 minutes to 6 hours.

By "delayed and controlled release" is meant release of the drug is delayed for an initial lag time after which time the drug is released in a controlled manner.

By "delayed and rapid release" is meant release of the drug is delayed for an initial lag time after which time the drug is released in a rapid manner.

By "release profile" is meant a profile provided by indicating the amount of an active agent released from a dosage form into an environment of use as a function of time.

By "zero-order release profile" is meant a release profile provided by the release of a constant amount per unit time of an active agent to an environment. By "pseudo-zero order release profile" is meant a release profile that approximates a zero-order release profile.

By "first order release profile" is meant a release profile provided by the release of a constant percentage per unit time of an initial active agent charge to an environment. By "pseudo-first order release profile" is meant a release profile that approximates a first order release profile.

The invention provides for the administration of venlafaxine in combination with an anti-Alzheimer's drug, such as memantine. Venlafaxine (VFX) is available as ELAFAX® (Gador, Argentina), EFEXOR® (Wyeth-Ayerst, Italy) and DOBUPAL® (Almirall-Prodesfarma, Spain) among others. Memantine (GA) is available as EBIXA (H. Lundeck A/S, Denmark), AXURA® (Merz Pharmaceuticals GmbH, Germany) and AKATINOL (Merz Pharmaceuticals GmbH, Germany and Phoenix, Argentina, under license) These compounds are in their free base, free acid, racemic, optically pure, diastereomeric and/or pharmaceutically acceptable salt forms. All such forms are considered within the scope of the present invention.

As used herein, "pharmaceutically acceptable salts" refer to derivatives of the disclosed compounds wherein the therapeutic compound is modified by making acid or base salts thereof. Examples of pharmaceutically acceptable salts include, but are not limited to, mineral or organic acid salts of the VFX or memantine. The pharmaceutically acceptable salts include the conventional non-toxic salts, for example, from non-toxic inorganic or organic acids. For example, such conventional non-toxic salts include those derived from inorganic acids such as hydrochloric, hydrobromic, sulfuric, sulfonic, sulfamic, phosphoric, nitric and the like; and the salts prepared from organic acids such as amino acids, acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. Lists of suitable salts are found in *Remington's Pharmaceutical Sciences,* 17th ed., Mack Publishing Company, Easton, PA, 1985, pg. 1418, the disclosure of which is hereby incorporated by reference.

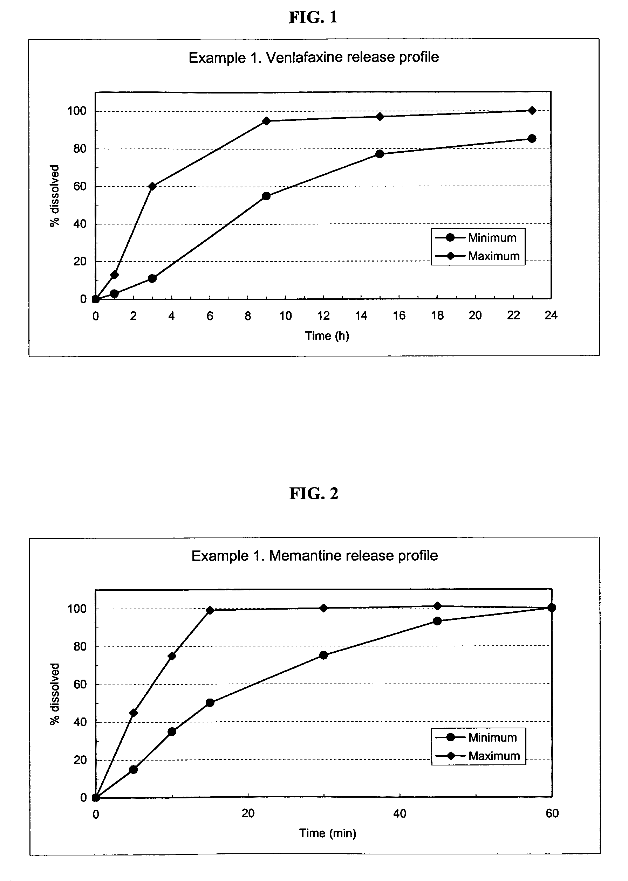

FIG. 1 depicts a range of venlafaxine *in vitro* release profiles for the osmotic device tablets described in Example 1. The venlafaxine release profile of this exemplary formulation is generally described as follows:

| Time (h) | Maximum percent released | Minimum percent released |
|---|---|---|
| 0 | 0 | 0 |
| 1 | 13 | 3 |
| 3 | 60 | 11 |
| 9 | 95 | 55 |
| 15 | 97 | 77 |
| 23 | 100 | 85 |

FIG 2. depicts a range of memantine *in vitro* release profiles for the osmotic device tablets described in Example 1. The profiles can be described as follows.

| Time (min) | Maximum percent released | Minimum percent released |
|---|---|---|
| 0 | 0 | 0 |
| 5 | 45 | 15 |
| 10 | 75 | 35 |
| 15 | 99 | 50 |
| 30 | 100 | 75 |
| 45 | 101 | 93 |
| 60 | 100 | 100 |

The values set forth in the above tables are approximate numbers. Depending upon the conditions of measurement as well as the assay used to determine those values, they may have a standard deviation of ± 2%, ± 5% or ± 10% of the indicated value.

The osmotic device generally provides the above-described plasma profile after administration of a single daily dose, i.e. acute dosing. The artisan of ordinary skill will understand that chronic daily dosing of the osmotic device will generally result in a relatively flat plasma profile over a 24-hour period for venlafaxine and optionally memantine, since a steady-state or equilibrium will be reached due to chronic administration. Steady-state levels of memantine are present in chronically treated patients; the half-life of memantine in humans is up to 100 hours. The serum levels of memantine with daily maintenance dose of 20 mg range from 0.5 to 1.0 µM.

The release profile of the drug delivery device of the invention is preferred as it provides a lower Cₘₐₓ and longer Tₘₐₓ while at the same time maintaining therapeutically effective levels thereof over an extended period of time.

Depending upon the disorder or disease being treated, the amounts of venlafaxine and memantine included in a dosage form may need to be varied. For example, a dosage form containing a first amount of VFX (venlafaxine) and a first amount of MEM (memantine) might be useful for treating symptoms associated with Alzheimer's disease; whereas a second dosage form containing a second amount of VFX and a second amount of MEM might be useful for treating symptoms associated with Parkinson's disease. The amount of each drug included in a single dosage form might be the same or different.

Depending upon the particular combination of ingredients used to prepare the controlled release device, it will generally provide an expected uniform release rate of the controlled release venlafaxine and an overall release profile resembling a pseudo-zero order, zero-order, pseudo-first order or first order release profile.

Tablet formulations of the invention provide effective levels of venlafaxine and memantine for at least a predetermined period of time. The tablets of the invention will generally provide therapeutically effective amounts of venlafaxine for a total time period of not less than 18 hours and not more than 30 hours, generally not less than 20 hours and not more than 24 hours, or not less than 22 hours. The controlled release core generally begins to release venlafaxine within about 2 hours after administration.

The external coating can be an immediately dissolving coating that dissolves in the buccal cavity or a rapidly dissolving coating that dissolves in the stomach, jejunum or duodenum. The rapid release coating will release all of the memantine within 3 hours after administration and preferably at least 75% of memantine within about 30 or about 45 minutes after administration. While memantine is released over a short period of time, the therapeutic benefit that it provides will last at least 8 hours and generally up to about 18-24 hours.

Those of ordinary skill in the art will appreciate that the particular amounts of venlafaxine and memantine used in the osmotic device will vary according to, among other things, the desired pharmacokinetic behavior in a mammal.

When a rapidly dissolving coat is used in the tablet formulations of the invention, the coat will generally comprise an inert and non-toxic material that is at least partially, and generally substantially completely, soluble or erodible in an environment of use. The rapidly dissolving coat will be soluble in the buccal cavity and/or upper GI tract, such as the stomach, duodenum, jejunum or upper small intestines. Exemplary materials are disclosed in U.S. Patents No. 4,576,604 and 4,673,405, and the text Pharmaceutical Dosage Forms: Tablets Volume I, Second Edition. (A. Lieberman. ed. 1989, Marcel Dekker, Inc.) the relevant disclosures of which are hereby incorporated by reference. In some embodiments, the rapidly dissolving coat will be soluble in saliva, gastric juices, or acidic fluids.

When a delayed release coat is used, the osmotic device of the invention may include an enteric coat that resists the action of gastric fluid and is soluble and/or erodible in intestinal juices, substantially pH neutral or basic fluids but for the most part insoluble in gastric juices or acidic fluids. A wide variety of polymeric materials are known to possess these various solubility properties. Such polymeric materials include, by way of example and without limitation, cellulose acetate phthalate (CAP), cellulose acetate trimelletate (CAT), poly(vinyl acetate) phthalate (PVAP), hydroxypropyl methylcellulose phthalate (HP), poly(methacrylate ethylacrylate) (1:1) copolymer (MA-EA), poly(methacrylate methylmethacrylate) (1:1) copolymer (MA-MMA), poly(methacrylate methylmethacrylate) (1:2) copolymer, Eudragit L-30-D™ (MA-EA, 1:1), Eudragit™ L-100-55™ (MA-EA, 1:1), hydroxypropyl methylcellulose acetate succinate (HPMCAS), Coateric™ (PVAP), Aquateric™ (CAP), AQUACOAT™ (HPMCAS), poly(vinylpyrrolidone)-vinyl acetate copolymer, such as the material supplied by BASF under its Kollidon VA64 trademark, mixed with magnesium stearate and other similar excipients and combinations thereof. The enteric coat can also comprise poly(vinyl pyrrolidone), such as the material supplied by BASF under its Kollidon K 30 trademark, and hydroxypropyl methylcellulose, which is supplied by Dow under its Methocel E-15 trademark. The materials can be prepared in solutions of having different concentrations of polymer according to the desired solution viscosity. For example, a 10% PN aqueous solution of Kollidon K 30 has a viscosity of about 5.5-8.5 cps at 20 °C, and a 2% PN aqueous solution of Methocel E-15 has a viscosity of about 13-18 cps at 20°C. The enteric coat can also comprise dissolution aids, stability modifiers, and bioabsorption enhancers.

When the enteric coat is intended to be dissolved, eroded or become detached from the rest of the device while in the colon, materials such as hydroxypropylcellulose, microcrystalline cellulose (MCC, Avicel™ from FMC Corp.), poly (ethylene - vinyl acetate) (60:40) copolymer (EVAC from Aldrich Chemical Co.), 2-hydroxyethylmethacrylate (HEMA), MMA, terpolymers of HEMA: MMA:MA synthesized in the presence of N,N'-bis(methacryloyloxyethyloxycarbonylamino) - azobenzene, azopolymers, enteric coated timed release system (Time Clock® from Pharmaceutical Profiles, Ltd., UK) and calcium pectinate can be used.

The enteric coat can comprise one or more materials that do not dissolve, disintegrate, or change their structural integrity in the stomach and during the period of time that the tablet resides in the stomach. Representative materials that keep their integrity in the stomach can comprise a member selected from the group consisting of (a) keratin, keratin sandarac-tolu, salol (phenyl salicylate), salol beta-naphthylbenzoate and acetotannin, salol with balsam of Peru, salol with tolu, salol with gum mastic, salol and stearic acid, and salol and shellac; (b) a member selected from the group consisting of formalized protein, formalized gelatin, and formalized cross-linked gelatin and exchange resins; (c) a member selected from the group consisting of myristic acid-hydrogenated castor oil-cholesterol, stearic acid-mutton tallow, stearic acid-balsam of tolu, and stearic acid-castor oil; (d) a member selected from the group consisting of shellac, ammoniated shellac, ammoniated shellac-salol, shellac-wool fat, shellac-acetyl alcohol, shellac-stearic acid-balsam of tolu, and shellac n-butyl stearate; (e) a member selected from the group consisting of abietic acid, methyl abictate, benzoin, balsam of tolu, sandarac, mastic with tolu, and mastic with tolu, and mastic with acetyl alcohol; (f) acrylic resins represented by anionic polymers synthesized from methacrylate acid and methacrylic acid methyl ester, copolymeric acrylic resins of methacrylic and methacrylic acid and methacrylic acid alkyl esters, copolymers of alkacrylic acid and alkacrylic acid alkyl esters, acrylic resins such as dimethylaminoethylmethacrylate-butylmethacrylate-methylmethacrylate copolymer of 150,000 molecular weight, methacrylic acid-methylmethacrylate 50:50 coploymer of 135,000 molecular weight, methacrylic acid-methylmethacrylate-30:70-copolymer of 135,000 mol. wt., methacrylic acid-dimethylaminoethyl-methacrylate-ethylacrylate of 750,000 mol. wt., methacrylic acid-methylmethacrylate-ethylacrylate of 1,000,000 mol. wt., and ethylacrylate-methylmethacrylate-ethylacrylate of 550,000 mol. wt; and, (g) an enteric composition comprising a member selected from the group consisting of cellulose acetyl phthalate, cellulose diacetyl phthalate, cellulose triacetyl phthalate, cellulose acetate phthalate, hydroxypropyl methylcellulose phthalate, sodium cellulose acetate phthalate, cellulose ester phthalate, cellulose ether phthalate, methylcellulose phthalate, cellulose ester-ether phthalate, hydroxypropyl cellulose phthalate, alkali salts of cellulose acetate phthalate, alkaline earth salts of cellulose acetate phthalate, calcium salt of cellulose acetate phthalate, ammonium salt of hydroxypropyl methylcellulose phthalate, cellulose acetate hexahydrophthalate, hydroxypropyl methylcellulose hexahydrophthalate, polyvinyl acetate phthalate diethyl phthalate, dibutyl phthalate, dialkyl phthalate wherein the alkyl comprises from 1 to 7 straight and branched alkyl groups, aryl phthalates, and other materials known to one or ordinary skill in the art.

The semipermeable membrane of the osmotic device is typically formed of a material that is substantially permeable to the passage of fluid from the environment of use to the core and substantially impermeable to the passage of active agent from the core. Many common materials known by those of ordinary skill in the art are suitable for this purpose. Exemplary materials are cellulose esters, cellulose diesters, cellulose triesters, cellulose ethers, cellulose ester-ether, cellulose acylate, cellulose diacylate, cellulose triacylate, cellulose acetate, cellulose diacetate, cellulose triacetate, cellulose acetate propionate, cellulose acetate butyrate and ethylcellulose. A preferred semipermeable membrane material is cellulose acetate, commercially available from Eastman Chemical Products. The semipermeable membrane can also contain flux enhancing agents which increase the volume of fluid imbibed into the core, such as sugar, mannitol, sucrose, sorbitol, sodium chloride, potassium chloride, polyethylene glycol (weight av. molecular weight 380-3700), propylene glycol, hydroxypropyl cellulose, hydroxypropyl methylcellulose and mixtures thereof. A preferred flux enhancer is PEG 400. The semipermeable membrane can also contain plasticizers. Suitable plasticizers for manufacturing the semipermeable membrane include sebacate, dibutylsebacate, adipate, azelate, enzoate, citrate, triethylcitrate, tributylcitrate, glyceroltributyrate, acetyltributylcitrate, acetyltriethylcitrate, stearate, isoebucate, citric acid esters, diethyloxalate, acetylated monoglyceride, oils such as olive, sesame and rape seed oil, and the like. A preferred plasticizer is triacetin. The ratio of the components and the thickness of the semipermeable membrane can be varied to alter permeability and ultimately the release profile of the osmotic device. Many suitable polymers include those disclosed in U.S. patent No. 4,814,183 and other references cited herein, the disclosures of which are hereby incorporated by reference.

The core formulation can contain osmotically effective compounds, osmotic agents, osmagents and osmopolymers, which build up the osmotic pressure and/or the physical forces that release the venlafaxine and/or memantine from the core. The osmagents can also aid in either the suspension or dissolution of the venlafaxine and/or memantine in the core. Exemplary osmagents include organic and inorganic compounds such as salts, acids, bases, chelating agents, sodium chloride, lithium chloride, magnesium chloride, magnesium sulfate, lithium sulfate, potassium chloride, sodium sulfite, calcium bicarbonate, sodium sulfate, calcium sulfate, calcium lactate, d-mannitol, urea, tartaric acid, fructose, raffinose, sucrose, alpha-d-lactose monohydrate, glucose, combinations thereof and other similar or equivalent materials which are widely known in the art. Osmopolymers suitable for manufacturing the core of the invention are hydrophilic polymers that swell or expands usually exhibiting a 2 to 50 fold volume increase. Examplary osmopolymers include hydroxypropyl methylcelluloses (viscosity from 3 to 100,000 cps, measured in 2% w/v solution); ethylcelluloses (viscosity from 3 to 110 cP, measured in 5% w/v solution); methylcelluloses (viscosity from 10 to 10,000 cP, measured in 2% w/v solution); hydroxypropylcelluloses (general average molecular weight of about 80,000 to 1,150,000); hydroxyethylcelluloses (viscosity from 2 to 21,000 cP, measured in 2% w/v solution); carboxymethylcelluloses (viscosity from 5 to 4,000 cP, measured in 1% w/v solution); poly(hydroxyalkyl methacrylate) having a molecular weight of from 30,000 to 5,000,000; water swellable polymers of N-vinyl lactams, polyacrylamides (Cyanamer.RTM), polyacrylic acid having a molecular weight of 80,000 to 200,000, poly(vinylpyrrolidone) having molecular weight of from 10,000 to 360,000; poly (alkylene) oxide that might include homopolymer of ethylene oxide having a weight average molecular weight of 100,000 to 6,000,000 (Polyox.RTM), propylene oxide and butylene oxide and copolymers of those, and the like and mixtures thereof. The core of the osmotic device tablet of the present invention will comprise venlafaxine, at least one pharmaceutically acceptable excipient and optionally one or more other materials. Generally, the tablet formulations will comprise about 0.1-99.9% by weight of venlafaxine in the uncoated tablet core.

The osmotic device of the invention can also comprise an acidifying agent, alkalizing agent, adsorbent, antioxidant, buffering agent, colorant, flavorant, sweetening agent, antiadherent, binder, diluent, direct compression excipient, disintegrant, glidant, lubricant, opaquant and/or polishing agents.

As used herein, the term "adsorbent" is intended to mean an agent capable of holding other molecules onto its surface by physical or chemical (chemisorption) means. Such compounds include, by way of example and without limitation, powdered and activated charcoal and other materials known to one of ordinary skill in the art.

As used herein, the term "antioxidant" is intended to mean an agent that inhibits oxidation and thus is used to prevent the deterioration of preparations by the oxidative process. Such compounds include, by way of example and without limitation, ascorbic acid, ascorbyl palmitate, butylated hydroxyanisole, butylated hydroxytoluene, hypophophorous acid, monothioglycerol, propyl gallate, sodium ascorbate, sodium bisulfite, sodium formaldehyde sulfoxylate and sodium metabisulfite and other materials known to one of ordinary skill in the art.

As used herein, the term "alkalizing agent" is intended to mean a compound used to provide alkaline medium for product stability. Such compounds include, by way of example and without limitation, ammonia solution, ammonium carbonate, diethanolamine, monoethanolamine, potassium hydroxide, sodium borate, sodium carbonate, sodium bicarbonate, sodium hydroxide, triethanolamine, and trolamine and others known to those of ordinary skill in the art.

As used herein, the term "acidifying agent" is intended to mean a compound used to provide an acidic medium for product stability. Such compounds include, by way of example and without limitation, acetic acid, amino acid, citric acid, fumaric acid and other alpha-hydroxy acids, such as hydrochloric acid, ascorbic acid, and nitric acid and others known to those of ordinary skill in the art.

As used herein, the term "buffering agent" is intended to mean a compound used to resist change in pH upon dilution or addition of acid or alkali. Such compounds include, by way of example and without limitation, potassium metaphosphate, potassium phosphate, monobasic sodium acetate and sodium citrate anhydrous and dihydrate and other materials known to one of ordinary skill in the art.

As used herein, the term "sweetening agent" is intended to mean a compound used to impart sweetness to a preparation. Such compounds include, by way of example and without limitation, aspartame, dextrose, glycerin, mannitol, saccharin sodium, sorbitol and sucrose and other materials known to one of ordinary skill in the art.

As used herein, the term " antiadherent" is intended to mean an agent which prevent the sticking of tablet formulation ingredients to punches and dies in a tableting machine during production. Such compounds include, by way of example and without limitation, magnesium stearate, talc, calcium stearate, glyceryl behenate, PEG, hydrogenated vegetable oil, mineral oil, stearic acid and other materials known to one of ordinary skill in the art.

As used herein, the term " binder" is intended to mean a substance used to cause adhesion of powder particles in table granulations. Such compounds include, by way of example and without limitation, acacia, alginic acid, carboxymethylcellulose sodium, poly(vinylpyrrolidone), compressible sugar (e.g., NuTab), ethylcellulose, gelatin, liquid glucose, methylcellulose, povidone and pregelatinized starch and other materials known to one of ordinary skill in the art.

When needed, binders may also be included in the tablets. Exemplary binders include acacia, tragacanth, gelatin, starch, cellulose materials such as methyl cellulose and sodium carboxy methyl cellulose, alginic acids and salts thereof, polyethylene glycol, guar gum, polysaccharide, bentonites, sugars, invert sugars, poloxamers (PLURONIC F68, PLURONIC F127), collagen, albumin, gelatin, cellulosics in nonaqueous solvents, combinations thereof and the like. Other binders include, for example, polypropylene glycol, polyoxyethylene-polypropylene copolymer, polyethylene ester, polyethylene sorbitan ester, polyethylene oxide, combinations thereof and other materials known to one of ordinary skill in the art.

As used herein, the term " diluent" or "filler" is intended to mean inert substances used as fillers to create the desired bulk, flow properties, and compression characteristics in the preparation of tablets and capsules. Such compounds include, by way of example and without limitation, dibasic calcium phosphate, kaolin, lactose, sucrose, mannitol, microcrystalline cellulose, powdered cellulose, precipitated calcium carbonate, sorbitol, and starch and other materials known to one of ordinary skill in the art.

As used herein, the term " direct compression excipient" is intended to mean a compound used in direct compression tablet formulations. Such compounds include, by way of example and without limitation, dibasic calcium phosphate (e.g., Ditab) and other materials known to one of ordinary skill in the art.

As used herein, the term " glidant" is intended to mean agents used in tablet and capsule formulations to reduce friction during tablet compression. Such compounds include, by way of example and without limitation, colloidal silica, cornstarch, talc, calcium silicate, magnesium silicate, colloidal silicon, silicon hydrogel and other materials known to one of ordinary skill in the art.

As used herein, the term " lubricant" is intended to mean substances used in tablet formulations to reduce friction during tablet compression. Such compounds include, by way of example and without limitation, calcium stearate, magnesium stearate, mineral oil, stearic acid, and zinc stearate and other materials known to one of ordinary skill in the art.

As used herein, the term " opaquant" is intended to mean a compound used to render a capsule or a tablet coating opaque. May be used alone or in combination with a colorant. Such compounds include, by way of example and without limitation, titanium dioxide and other materials known to one of ordinary skill in the art.

As used herein, the term " polishing agent" is intended to mean a compound used to impart an attractive sheen to coated tablets. Such compounds include, by way of example and without limitation, carnauba wax, and white wax and other materials known to one of ordinary skill in the art.

As used herein, the term " disintegrant" is intended to mean a compound used in solid dosage forms to promote the disruption of the solid mass into smaller particles which are more readily dispersed or dissolved. Exemplary disintegrants include, by way of example and without limitation, starches such as corn starch, potato starch, pre-gelatinized and modified starches thereof, sweeteners, clays, such as bentonite, microcrystalline cellulose(e.g., Avicel), carboxymethylcellulose calcium, cellulose polyacrilin potassium (e.g., Amberlite), alginates, sodium starch glycolate, gums such as agar, guar, locust bean, karaya, pectin, tragacanth and other materials known to one of ordinary skill in the art.

As used herein, the term "colorant" is intended to mean a compound used to impart color to solid (e.g., tablets) pharmaceutical preparations. Such compounds include, by way of example and without limitation, FD&C Red No. 3, FD&C Red No. 20, FD&C Yellow No. 6, FD&C Blue No. 2, D&C Green No. 5, D&C Orange No. 5, D&C Red No. 8, caramel, and ferric oxide, red, other F.D. & C. dyes and natural coloring agents such as grape skin extract, beet red powder, beta-carotene, annato, carmine, turmeric, paprika, and other materials known to one of ordinary skill in the art. The amount of coloring agent used will vary as desired.

As used herein, the term "flavorant" is intended to mean a compound used to impart a pleasant flavor and often odor to a pharmaceutical preparation. Exemplary flavoring agents or flavorants include synthetic flavor oils and flavoring aromatics and/or natural oils, extracts from plants, leaves, flowers, fruits and so forth and combinations thereof. These may also include cinnamon oil, oil of wintergreen, peppermint oils, clove oil, bay oil, anise oil, eucalyptus, thyme oil, cedar leave oil, oil of nutmeg, oil of sage, oil of bitter almonds and cassia oil. Other useful flavors include vanilla, citrus oil, including lemon, orange, grape, lime and grapefruit, and fruit essences, including apple, pear, peach, strawberry, raspberry, cherry, plum, pineapple, apricot and so forth. Flavors which have been found to be particularly useful include commercially available orange, grape, cherry and bubble gum flavors and mixtures thereof. The amount of flavoring may depend on a number of factors, including the organoleptic effect desired. Flavors will be present in any amount as desired by those of ordinary skill in the art. Particular flavors are the grape and cherry flavors and citrus flavors such as orange.

The present osmotic device can also employ one or more commonly known surface active agents or cosolvents that improve wetting or disintegration of the tablet core or layers.

Plasticizers can also be included in the osmotic device to modify the properties and characteristics of the polymers used in the coats or core of the osmotic device. As used herein, the term "plasticizer" includes all compounds capable of plasticizing or softening a polymer or binder used in invention. The plasticizer should be able to lower the melting temperature or glass transition temperature (softening point temperature) of the polymer or binder. Plasticizers, such as low molecular weight PEG, generally broaden the average molecular weight of a polymer in which they are included thereby lowering its glass transition temperature or softening point. Plasticizers also generally reduce the viscosity of a polymer. It is possible the plasticizer will impart some particularly advantageous physical properties to the osmotic device of the invention.

Plasticizers useful in the invention can include, by way of example and without limitation, low molecular weight polymers, oligomers, copolymers, oils, small organic molecules, low molecular weight polyols having aliphatic hydroxyls, ester-type plasticizers, glycol ethers, poly(propylene glycol), multi-block polymers, single block polymers, low molecular weight poly(ethylene glycol), citrate ester-type plasticizers, triacetin, propylene glycol and glycerin. Such plasticizers can also include ethylene glycol, 1,2-butylene glycol, 2,3-butylene glycol, styrene glycol, diethylene glycol, triethylene glycol, tetraethylene glycol and other poly(ethylene glycol) compounds, monopropylene glycol monoisopropyl ether, propylene glycol monoethyl ether, ethylene glycol monoethyl ether, diethylene glycol monoethyl ether, sorbitol lactate, ethyl lactate, butyl lactate, ethyl glycolate, dibutylsebacate, acetyltributylcitrate, triethyl citrate, acetyl triethyl citrate, tributyl citrate and allyl glycolate. All such plasticizers are commercially available from sources such as Aldrich or Sigma Chemical Co. It is also contemplated and within the scope of the invention, that a combination of plasticizers may be used in the present formulation. The PEG based plasticizers are available commercially or can be made by a variety of methods, such as disclosed in Poly(ethylene glycol) Chemistry: Biotechnical and Biomedical Applications (J.M. Harris, Ed.; Plenum Press, NY) the disclosure of which is hereby incorporated by reference.

The osmotic device of the invention can also include oils, for example, fixed oils, such as peanut oil, sesame oil, cottonseed oil, corn oil and olive oil; fatty acids, such as oleic acid, stearic acid and isotearic acid; and fatty acid esters, such as ethyl oleate, isopropyl myristate, fatty acid glycerides and acetylated fatty acid glycerides. It can also be mixed with alcohols, such as ethanol, isopropanol, hexadecyl alcohol, glycerol and propylene glycol; with glycerol ketals, such as 2,2-dimethyl-1,3-dioxolane-4-methanol; with ethers, such as poly(ethyleneglycol) 450, with petroleum hydrocarbons, such as mineral oil and petrolatum; with water, or with mixtures thereof; with or without the addition of a pharmaceutically suitable surfactant, suspending agent or emulsifying agent.

Soaps and synthetic detergents may be employed as surfactants and as vehicles for detergent compositions. Suitable soaps include fatty acid alkali metal, ammonium, and triethanolamine salts. Suitable detergents include cationic detergents, for example, dimethyl dialkyl ammonium halides, alkyl pyridinium halides, and alkylamine acetates; anionic detergents, for example, alkyl, aryl and olefin sulfonates, alkyl, olefin, ether and monoglyceride sulfates, and sulfosuccinates; nonionic detergents, for example, fatty amine oxides, fatty acid alkanolamides, and poly(oxyethylene)-block-poly(oxypropylene) copolymers; and amphoteric detergents, for example, alkyl β-aminopropionates and 2-alkylimidazoline quaternary ammonium salts; and mixtures thereof.

Various other components, not otherwise listed above, can be added to the present formulation for optimization of a desired active agent release profile including, by way of example and without limitation, glycerylmonostearate, nylon, cellulose acetate butyrate, d, 1-poly(lactic acid), 1,6 - hexanediamine, diethylenetriamine, starches, derivatized starches, acetylated monoglycerides, gelatin coacervates, poly (styrene - maleic acid) copolymer, glycowax, castor wax, stearyl alcohol, glycerol palmitostearate, poly(ethylene), poly(vinyl acetate), poly(vinyl chloride), 1,3 - butylene-glycoldimethacrylate, ethyleneglycoldimethacrylate and methacrylate hydrogels.

It should be understood, that compounds used in the art of pharmaceutical formulation generally serve a variety of functions or purposes. Thus, if a compound named herein is mentioned only once or is used to define more than one term herein, its purpose or function should not be construed as being limited solely to that named purpose(s) or function(s).

The osmotic devices of the invention can assume any shape or form known in the art of pharmaceutical sciences. The device of the invention can be a pill, sphere, tablet, bar, plate, paraboloid of revolution, ellipsoid of revolution or the like. The osmotic device can also include surface markings, cuttings, grooves, letters and/or numerals for the purposes of decoration, identification and/or other purposes.

The tablets of the invention can be prepared according to the methods disclosed herein or those well known in the art, more specifically according to the methods disclosed in the disclosure incorporated herein by reference. The external coat can be applied as a compression coating, but it is generally applied as a sprayed coating. The sprayed coating is thinner and lighter than the compression coating, and an osmotic device including the sprayed on external coating is, therefore, smaller than a similar osmotic device having a compression coat. A smaller size osmotic device generally results in increased patient compliance in taking the osmotic device and is therefore advantageous.

The osmotic devices of the invention can be coated with a finish coat as is commonly done in the art to provide the desired shine, color, taste or other aesthetic characteristics. Materials suitable for preparing the finish coat are well known in the art and found in the disclosures of many of the references cited and incorporated by reference herein.

The osmotic device of the invention comprises at least one passageway (pore, hole, or aperture) that communicates the exterior of the semipermeable wall with the core of the device. The passageway can be formed according to any of the known methods of forming passageways in a semipermeable membrane. Such methods include, for example, 1) drilling a hole through the semipermeable membrane with a bit or laser; 2) including a water soluble material within the composition that forms the semipermeable membrane such that a pore forms when the osmotic device is in an aqueous environment of use; 3) punching a hole through the semipermeable membrane; or 4) employing a tablet punch having a pin to punch a hole through the semipermeable lamina. The passageway can pass through the semipermeable wall and one or more of any other lamina coated onto the semipermeable membrane or between the semipermeable membrane and the core. The passageway(s) can be shaped as desired. In some embodiments, the passageway is laser drilled and is shaped as an oval, ellipse, slot, slit, cross or circle.

Methods of forming passageways in semipermeable membranes of osmotic devices are disclosed in U.S. Patents No. 4,088,864 to Theeuwes et al., No. 4,016,880 to Theeuwes et al., No. 3,916,899 to Theeuwes et al., No. 4,285,987 to Ayer et al., No. 4,783,337 to Wong et al., No. 5,558,879 to Chen et al., No. 4,801,461 to Hamel et al., No. 3,845,770 to Theeuwes et al., and U.S. Pregrant Publication No. 20030189030 to Faour, the disclosures of which are hereby incorporated by reference.

The preformed passageway, e.g., one made by mechanical means, is formed after the semipermeable membrane is applied to the core. It can be formed either before or after the inert water soluble coat and/or drug-containing external coat is applied to the semipermeable membrane.

The advantages of the present system over known systems for administering venlafaxine in combination with memantine are improved therapeutic benefit, improved clinical benefit, simplified manufacturing, and increased patient compliance. Moreover, the present formulation will provide an enhanced therapeutic effect when compared to the administration of venlafaxine alone.

By administration of the venlafaxine in a controlled release fashion and the memantine in a rapid release fashion, the osmotic device unexpectedly provides an improved pharmacological profile including reduced side effects, lower drug requirement and/or enhanced therapeutic benefit as compared to other known methods or dosage forms. The dosage form can be administered to treat or ameliorate one or more symptoms associated with Alzheimer's disease, Parkinson's disease, Age Associated Memory Impairment, or other neurological diseases or disorders such as dementia, vascular dementia, HIV dementia, multiple sclerosis, drug dependence, epilepsy diabetic neuropathy, neuropathic pain and chronic pain. The dosage form can be administered once or twice per day; although, once per day administration is preferred.

Another embodiment of the invention provides for the administration of venlafaxine and memantine to a subject, wherein the drugs are administered sequentially, simultaneously or in an overlapping manner.

Sequential administration of the drugs means that one drug is delivered completely or substantially completely and then the other drug is delivered. Sequential administration can be achieved by administration of a dosage form comprising VFX (or MEM) and followed by administration of another dosage form comprising MEM (or VFX, respectively). If two separate dosage forms are used, they can have the same or different drug release profiles. If they have the same release profile, the dosage forms are administered one after the other, generally in spaced apart periods of time. If the dosage forms are administered at the same time, one dosage form will release drug first and the other dosage form will release drug after a delay period. Alternatively, sequential administration is achieved by the administration of a single dosage form comprising both drugs, wherein the first drug is released substantially completely and then the second drug is released after an initial delay period. For example, memantine is administered in immediate or rapid release form and venlafaxine is administered in controlled, extended or sustained release form such that release of VFX begins after a delay period such that most or all of the MEM has been released.

Simultaneous administration of the drugs means that both drugs are delivered at about the same time. Simultaneous administration can be achieved by administration of separate dosage forms that release drug according to substantially the same release profile, wherein the dosage forms are administered one immediately after the other or one within a short period of time after the other or both at the same time. Simultaneous administration can also be achieved by administration of a single dosage form comprising both drugs, wherein each drug is released according to substantially the same release profile. For example, VFX and MEM are included in a solid dosage form and the drugs are released simultaneously therefrom in an environment of use. Or for example, the VFX and MEM are included in a liquid dosage form and injected into a subject.

Overlapping administration of the drugs means that the drugs are delivered such that delivery of the first drug begins, then delivery of the second drug begins while delivery of the first drug is still ongoing. Delivery of the first drug can complete either before or after delivery of the second drug is complete. An exemplary solid dosage form provides a rapid release of the first drug and a controlled release of the second drug while release of the first drug is ongoing, then release of the first drug completes and release of the second drug continues until completion.

The clinical benefit provided by treatment of a disorder with the combination of VFX and MEM is at least additive. In one embodiment, the clinical benefit is synergistic. A synergistic clinical benefit can be any of the following:
1- an improved clinical benefit observed when a first drug (such as VFX) is administered at a therapeutic dose and a second drug (such as MEM) is administered at a sub-therapeutic dose such that the observed clinical benefit provides an improvement over administration of VFX alone at a therapeutic dose;
2- an improved clinical benefit observed when a first drug (such as MEM) is administered at a therapeutic dose and a second drug (such as VFX) is administered at a sub-therapeutic dose such that the observed clinical benefit provides an improvement over administration of MEM alone at a therapeutic dose;
   or
3- a clinical benefit observed when MEM is administered at a sub-therapeutic dose
and VFX is administered at a sub-therapeutic dose.

The procedures of Examples 2, 3, and 4 below provide a method of establishing the presence of an improved clinical benefit (either an additive clinical benefit or a synergistic clinical benefit) obtained by the administration of VFX and MEM to a subject. In the procedures of Examples 2 and 3, a reduction in scopolamine impairment of memory is indicative of cognitive enhancement. In the procedures of Example 4 an increase of the latency is indicative of improved retention performance. The results of the studies indicate that the administration of VFX and MEM to a subject in need of such treatment provides the improved clinical benefit. In one embodiment, the improved clinical benefit results from the simultaneous administration of both drugs. In another embodiment, the improved clinical benefit results from the sequential administration of both drugs. In yet another embodiment, the improved clinical benefit results from the overlapping administration of both drugs.

The following examples should not be considered exhaustive, but merely illustrative of only a few of the many embodiments contemplated by the present invention. The methods described herein can be followed to prepare osmotic devices according to the invention.

### EXAMPLE 1

The following procedure is used to prepare multi-layered osmotic device tablets containing venlafaxine (37.5, 75 and 150 mg strength) in the core and memantine (10, 20, 30 and 40 mg strength) in a drug-containing external coat of the osmotic device. The venlafaxine is released in a controlled manner and the memantine is released in a rapid manner. The osmotic device tablets contain the following ingredients in the amounts indicated.

| **INGREDIENT** | **AMOUNT (mg)** | | | | | | | |
|---|---|---|---|---|---|---|---|---|
| **Venlafaxine Strength ⇒** | 37.5 | 37.5 | 37.5 | 37.5 | 75 | 75 | 150 | 150 |
| **Memantine Strength ⇒** | 10 | 20 | 30 | 40 | 10 | 40 | 10 | 40 |

| **CORE** | | | | | | | | |
|---|---|---|---|---|---|---|---|---|
| Venlafaxine Hydrochloride | 42.43 | 42.43 | 42.43 | 42.43 | 84.86 | 84.86 | 169.72 | 169.72 |
| Mannitol | 25.00 | 25.00 | 25.00 | 25.00 | 50.00 | 50.00 | 100.00 | 100.00 |
| Povidone k-90 | 3.50 | 3.50 | 3.50 | 3.50 | 7.00 | 7.00 | 14.00 | 14.00 |
| Polyethylene Glycol 400 | 2.50 | 2.50 | 2.50 | 2.50 | 5.00 | 5.00 | 10.00 | 10.00 |
| Cellulose Microcrystalline | 14.57 | 14.57 | 14.57 | 14.57 | 29.14 | 29.14 | 58.28 | 58.28 |
| Colloidal Silicon Dioxide | 0.50 | 0.50 | 0.50 | 0.50 | 1.00 | 1.00 | 2.00 | 2.00 |
| Magnesium Stearate | 1.50 | 1.50 | 1.50 | 1.50 | 3.00 | 3.00 | 6.00 | 6.00 |
| Purified water | 15.00 | 15.00 | 15.00 | 15.00 | 30.00 | 30.00 | 60.00 | 60.00 |

| **COATING A** | | | | | | | | |
|---|---|---|---|---|---|---|---|---|
| Cellulose Acetate 398 | 7.88 | 7.88 | 7.88 | 7.88 | 15.77 | 15.77 | 31.54 | 31.54 |
| Polyethylene Glycol 400 | 0.42 | 0.42 | 0.42 | 0.42 | 0.83 | 0.83 | 1.66 | 1.66 |
| Acetone | 130.37 | 130.37 | 130.37 | 130.37 | 260.74 | 260.74 | 521.48 | 521.48 |

| **COATING B** | | | | | | | | |
|---|---|---|---|---|---|---|---|---|
| Memantine Hydrochloride | 12.03 | 24.07 | 36.10 | 48.13 | 12.03 | 48.13 | 12.03 | 48.13 |
| HPMC 2910 | 1.47 | 2.93 | 4.40 | 5.87 | 3.97 | 15.87 | 6.97 | 27.87 |
| Crospovidone | 0.50 | 1.00 | 1.50 | 2.00 | 2.00 | 8.00 | 2.50 | 10.00 |
| Polyethylene Glycol 400 | 1.00 | 2.00 | 3.00 | 4.00 | 2.00 | 8.00 | 3.50 | 14.00 |
| Acetone | 120.00 | 240.00 | 360.00 | 480.00 | 160.00 | 640.00 | 200.00 | 800.00 |

| **COATING C** | | | | | | | | |
|---|---|---|---|---|---|---|---|---|
| Opadry 1 | 10.00 | 10.00 | 10.00 | 10.00 | 15.00 | 15.00 | 20.00 | 20.00 |
| Purified Water | 100.00 | 100.00 | 100.00 | 100.00 | 150.00 | 150.00 | 200.00 | 200.00 |

The core composition is prepared by placing venlafaxine, mannitol, cellulose microcrystalline and half the quantity of colloidal silicon dioxide in a high shear mixer and mixing for 3 minutes. The granulation process is initiated by the gradual addition of a granulating fluid comprising polyethylene glycol 400, povidone, and purified water to the mixer with continuous mixing to produce a wet blend. The wet blend is granulated and dried at 40-50°C for 15 minutes in a fluid bed to remove the granulating fluid. The dry granules are screened through a mesh screen for size reduction. The screened granules are mixed with the rest of colloidal silicon dioxide, that has been previously passed through a 60 mesh screen, and mixed with magnesium stearate, that has been previously passed through a 60 mesh screen. This final blend is tabletted to provide the cores.

A first composition to cover the coated cores is prepared as follows. Cellulose acetate 398 and polyethylene glycol 400 are added to acetone and mixed thoroughly to form a polymer mixture. This polymer mixture is sprayed onto the tablets in a perforated pan coater to form semipermeable membrane coated cores. A 0.5 mm hole is drilled through the coating to provide perforated cores. This first coating composition can also be manufactured with the ingredients in the amounts indicated in the table below as follows. Cellulose acetate 398, cellulose acetate 320 and polyethylene glycol 400 are added to a blend of methylene chloride, methanol and acetone, and mixed thoroughly to form a polymer mixture that is sprayed onto the tablets. A 0.5 mm hole is drilled through the coating.

| **COATING A** | | | | | | | | |
|---|---|---|---|---|---|---|---|---|
| Cellulose Acetate 398 | 8.02 | 8.02 | 8.02 | 8.02 | 16.03 | 16.03 | 32.06 | 32.06 |
| Cellulose Acetate 320 | 2.11 | 2.11 | 2.11 | 2.11 | 4.22 | 4.22 | 8.44 | 8.44 |
| PEG 400 | 0.52 | 0.52 | 0.52 | 0.52 | 1.05 | 1.05 | 2.10 | 2.10 |
| Methylene Chloride | 5.91 | 5.91 | 5.91 | 5.91 | 11.82 | 11.82 | 23.64 | 23.64 |
| Methanol | 120.07 | 120.07 | 120.07 | 120.07 | 140.14 | 140.14 | 280.28 | 280.28 |
| Acetone | 50.00 | 50.00 | 50.00 | 50.00 | 100.00 | 100.00 | 200.00 | 200.00 |
| Purified water | 21.10 | 21.10 | 21.10 | 21.10 | 42.20 | 42.20 | 84.40 | 84.40 |

A second composition to cover the perforated cores is prepared as follows. Memantine HC1, HPMC 2910, crospovidone and polyethylene glycol 400 are added to the acetone to form a polymer mixture. This polymer mixture is sprayed onto the tablets in a perforated pan coater to obtain film-coated tablets.

A finish coat comprising Opadry in purified water is applied onto the film-coated tablets to obtain the multi-layered osmotic device tablets.

### EXAMPLE 2

The following procedure is used to evaluate the combined use of venlafaxine and memantine for at least additive or synergistic activity in the scopolamine-induced memory impairment in the eight-arm radial maze test.

### Materials and Methods

### Animals

Male Sprague-Dawley rats (Bioterio Central, Facultad de Farmacia y Bioquimica, Universidad de Buenos Aires) weighing 200-250 g on arrival are used. Rats are housed 4 per cage, with standard laboratory food and water available *ad libitum* in a room maintained at 22± 2 °C, humidity 60%, with a 12 hour light/dark cycle with lights on at 8:00 AM. One week after arrival animals are housed individually and deprived of food in order to decrease its body weight by 85%. All experiments will be performed between 9 a.m. and 12 a.m.

### Eight-arm radial maze apparatus

The apparatus is elevated to a height of 50 cm and is composed of an octagonal central platform surrounded by 8 arms radiating away from the center, equidistant from one another. Each arm is 40 cm length, 10 cm wide and 34 cm high. At the distal end of each arm, there is a little food cup. A clear plexiglass cylinder encloses the animal in the center portion of the apparatus prior to the start of each session, and is removed 10 seconds after the animal is put in the maze. Each arm of the maze is equipped with 3 sets of photocells, used to track the movement of the rat in the apparatus. Photocells are interfaced to a computer with an in-house program for compilation and storage of the data. An automated dispenser, connected with the photocell near the end of each arm, delivers two 45 mg chocolate pellets (BIO-SERV) in the food cups the first time the animal crosses the photocell in a given session. The apparatus is located in a sound attenuated testing room next to the housing one, with 4 black and white geometric posters surrounding it in order to provide visual cues. During all training and testing procedures, white noise was audible.

### Training and testing procedures

The training procedure consists of consecutive daily sessions lasting 10 minutes. A 10 second delay is imposed between the time the rat is placed in the center portion of the apparatus and when the cylinder is raised to begin the session. During the first day, food-restricted pairs of rats are placed on the maze for 10 minutes with 45 mg chocolate food pellets scattered throughout the 8 arms of the maze. Day 2 each rat is placed individually on the maze for a 10 minute period, with pellets scattered from the middle photocell to the food cup of each arm. Day 3, each rat is placed on the maze for a 10 minute period, with food pellets located only in and around the food cups in each arm. On day 4, each rat is allowed 10 minutes to collect two pellets from the food cup at the end of each arm. Reentry into an arm is considered an error. Rats are trained daily in this manner until they achieve criterion performance with 2 or less errors on three consecutive days of training. Total habituation and training time lasts approximately 3 weeks. Trained rats are used in the test experiments.

### Test experiments

Thirty minutes before the test, trained animals are injected with the drugs (as described below). When the test begins, rats are placed at the center of the maze and are allowed to explore for 10 min. An error is computed when the animal re-enters an arm of the maze. Results are expressed as total number of errors.

### Drug preparation

Scopolamine hydrobromide (Sigma-Aldrich Co.), venlafaxine hydrochloride and memantine hydrochloride are prepared in saline solution and administered intraperitoneally in a volume of 1 ml/kg 30 minutes before the test experiment.

### Drug Treatments and statistical design

### a) Identification of drugs doses not affecting spontaneous locomotor activity

Performance in the radial maze may be nonspecifically influenced by the drugs, which may increase or decrease locomotor activity by stimulant or sedating actions. Therefore, separate experiments performed with naïve (e.g. not trained) animals are performed. Different groups of rats are injected with scopolamine (doses ranging from 0.01 to 1 mg/kg), venlafaxine (doses ranging from 0.3 to 10 mg/kg) and memantine (doses ranging from 1 to 100 mg/kg). Thirty minutes after the injection rats are individually placed in an open field apparatus (Panlab Digiscan) and locomotor activity is recorded. Comparative groups injected with saline are tested as controls. Results, expressed as mean ± SEM of beams crossed in 10 minutes are compared by ANOVA test followed by Dunnett's test. Only doses not significantly modifying locomotor activity are used in subsequent experiments.

### b) Identification of scopolamine impairing dose

In order to identify the memory impairing dose of scopolamine, previously trained rats are injected with scopolamine (with the doses obtained in experiment described in *a)*) or saline. Comparisons between control (saline group) and scopolamine treated rats are calculated by ANOVA test followed by Dunnett's test. The dose producing the highest impairment is used thereafter.

### c) Venlafaxine and memantine effect on scopolamine-induced memory impairment.

Trained rats are injected with scopolamine (dose identified in *b)*) in combination with venlafaxine (doses ranging from 0.3 to 10 mg/kg, providing no nonspecific effect on locomotor activity has been observed in *a)*) or memantine (doses ranging from 3 to 100 mg/kg, providing no nonspecific effect on locomotor activity has been observed in *a)*)*.* Control animals are injected with scopolamine and saline. Thirty minutes after the injection animals are tested in the radial maze apparatus. Results are expressed as mean ± SEM of the errors and a dose-effect curve is performed. ED₅₀ is calculated both for venlafaxine and for memantine.

### d) Additive effect of venlafaxine and memantine on scopolamine-induced memory impairment.

To establish the at least additive effect of venlafaxine and memantine on scopolamine-induced memory impairment, trained animals are divided into five groups: control, scopolamine, scopolamine+venlafaxine (ED₅₀ found in *c)*), scopolamine+memantine (ED₅₀ found in *c)*) and scopolamine+memantine+venlafaxine. To achieve high experimental efficiency with the least amount of animals, repeated measures are designed. Five experimental sessions with two animals per group are performed. Each treatment follows every other treatment the same number of times. Results, expressed as mean of number of errors ± SEM, are compared using ANOVA, followed by Bonferroni test.

### EXAMPLE 3

The following procedure is used to evaluate the combined use of venlafaxine and memantine for at least additive or synergistic activity in the scopolamine-induced memory impairment in the one trial step-through inhibitory avoidance test.

### Materials and Methods

### Animals

Male Sprague-Dawley rats (Bioterio Central, Facultad de Farmacia y Bioquimica, Universidad de Buenos Aires) weighing 200-250 g on arrival are used. Rats are housed 4 per cage, with standard laboratory food and water available *ad libitum* in a room maintained at 22± 2 °C, humidity 60%, with a 12-hour light/dark cycle with lights on at 8:00 AM.

### Inhibitory avoidance apparatus

The inhibitory avoidance box consists of two compartments (20 x 30 x 26-cm width, length, height each) connected by a door (10 x 10 cm). One of the compartments is brightly illuminated and the other is dark. The apparatus is located in a sound-attenuated room and is interfaced with a computer with an ad-hoc program which allows to automatically register and store the data of each experiment.

### Habituation procedure

Only one habituation session is performed in which each animal is first gently placed in the dark compartment for 5 min and returned to home cage for another 5 min. The animals are then gently placed in the light compartment, and the latency to enter the dark compartment with all four feet will be measured in seconds. Animals with a step-though latency that is longer than 20 s, in the habituation session, go through the previous habituation procedures several times, with 5 min between trials, until they enter the dark compartment in less than 20 s. Animals entering the dark compartment in less than 4 s are considered hyperactive and therefore are excluded from the experiment. Such excluded animals are replaced by other naïve ones. The habituation session is performed on these naïve animals for the purpose of reaching equal number of animals with latencies between 4 and 20 s in each group.

### Training procedure

This behavioral test is based in the innate preference of rodents for dark instead of lighted environment. The training and test trials are performed between 9 a.m. and 12 a.m. In the training trial, and thirty minutes after the injection of saline or the experimental drugs, animals are placed into the bright compartment. The door separating the compartments is opened 30 seconds later, and the latency to enter the dark compartment is measured. After the rat has entered the dark compartment the door is closed and a foot shock is delivered (0.5 mA, 2 s). Immediately after, the rat is removed from the apparatus and is returned to its home cage.

### Test experiments

Testing trial takes place 24 hours after the training one. Animals are again placed into the bright compartment; 30 seconds later the door is opened, and the latency (seconds) to re-enter the dark compartment is measured. The testing trial is finished after 180 seconds if the animal remains in the bright compartment. No foot shock is given during the tests session. Results are expressed as mean ± SEM of the latency (seconds) to enter the dark compartment and the differences between experimental and control values are compared.

### Drug preparation

Scopolamine hydrobromide (Sigma-Aldrich Co.), venlafaxine hydrochloride and memantine hydrochloride are prepared in saline solution and administered intraperitoneally in a volume of 1 ml/kg 30 minutes before the training experiment.

### Drug Treatments and statistical design

### a) Identification of the doses of scopolamine, venlafaxine and memantine not affecting locomotor activity

Performance in the inhibitory avoidance test may be nonspecifically influenced by the experimental drugs, which may increase or decrease locomotor activity by stimulant or sedating actions. Therefore, experiments with separates group of animals is performed. Different groups of rats are injected with scopolamine (doses ranging from 0.01 to 1 mg/kg), venlafaxine (doses ranging from 0.3 to 10 mg/kg) or memantine (doses ranging from 1 to 100 mg/kg). Thirty minutes after the injection rats are individually placed in an open field apparatus (Panlab Digiscan) and locomotor activity is recorded. Comparative groups injected with saline are tested as controls. Results, expressed as mean ± SEM of beams crossed in 5 minutes, are compared with those obtained in the saline groups by ANOVA test followed by Dunnet's test. Only doses not significantly modifying locomotor activity are used in subsequent experiments.

### b) Identification of scopolamine impairing dose

In order to identify the memory impairing dose of scopolamine, previously habituated rats are injected with scopolamine (with the doses obtained in experiment described in a)) or saline. The step-through inhibitory avoidance experiment is performed and results, expressed as mean ± SEM of the latency to cross to the dark compartment on the test day are recorded. Comparisons between control (saline group) and scopolamine treated rats are calculated by ANOVA test followed by Dunnett's test. The dose producing the highest impairment is used thereafter.

### c) Venlafaxine and memantine effect on scopolamine-induced memory impairment.

Habituated rats are injected with scopolamine (dose identified in b)) and venlafaxine (doses ranging from 0.3 to 10 mg/kg, providing no nonspecific effect on locomotor activity has been observed in a)) or memantine (doses ranging from 3 to 100 mg/kg, providing no nonspecific effect on locomotor activity has been observed in a)). Control animals receive scopolamine and saline. Thirty minutes after the injection, animals are trained in the inhibitory avoidance apparatus as previously described. Results are expressed as mean ± SEM of the latency (seconds) to re-enter the dark compartment on the test day, and a dose-effect curve is performed. ED₅₀ is calculated both for venlafaxine and memantine.

### d) Additive effect of venlafaxine and memantine on scopolamine-induced memory impairment.

To establish the at least additive or synergistic effect of the combination of venlafaxine and memantine on scopolamine-induced memory impairment, habituated animals are divided into five groups: control, scopolamine, scopolamine + venlafaxine (ED₅₀ found in c)), scopolamine + memantine (ED₅₀ found in c)) and scopolamine + memantine + venlafaxine. Thirty minutes after the injection animals are trained in the inhibitory avoidance apparatus as previously described. To achieve high experimental efficiency with the least amount of animals, repeated measures are designed. Five experimental sessions with two animals per group are performed. Each treatment follows every other treatment the same number of times. Results, expressed as mean ± SEM of latency (seconds) to re-enter the dark compartment on the test day, are compared using ANOVA followed by Bonferroni test.

### EXAMPLE 4

The following procedure is used to evaluate the combined use of venlafaxine and memantine for at least additive or synergistic activity in the one trial step-through inhibitory avoidance test.

### Materials and Methods

### Experimental Subjects

CF-1 male mice (FUNDACAL, Argentina) are used (age: 60-70 days; weight: 25-30 g). They are individually caged and remain singly housed throughout the experimental procedures. The mice are kept in a climate controlled animal room (21-23 °C) maintained on a 12-h light/dark cycle (lights on at 06:00 h), with *ad libitum* access to dry food and tap water. Experiments are carried out in accordance with the National Institute of Health Guide for the Care and Use of Laboratory Animals (NIH Publication No. 80-23/96), and local regulations. All efforts are made to minimize animal suffering and to reduce the number of animals used.

### Inhibitory Avoidance Tasks

### Step-through

Inhibitory avoidance behavior is studied in a one-trial learning, step-through type situation (Neurobiol Learn Mem (2000) 74: 217-228), which utilizes the natural preference of mice for a dark environment. The apparatus consists of a dark compartment (20x20x15 cm) with a stainless-steel grid floor and a small (5x5 cm) illuminated platform attached to its front center. The mice are not habituated to the dark compartment before the learning trial. During training each mouse is placed on the illuminated platform and received a footshock (0.8 mA, 50 Hz, 1 s) as it stepped into the dark compartment. The drugs are administered immediately after training. Venlafaxine is administered in doses ranging from 0.3 to 10 mg/kg. Memantine is administered in doses ranging from 3 to 100 mg/kg.

Forty eight hours after administration of the drugs the retention test is performed. Thus, each mouse is placed on the platform again and the step-through latency is recorded. If a mouse failed to cross within 300 s (ceiling score), the retention test is terminated and the mouse is assigned a score of 300 s. In the retention test session the footshock is omitted.

### Step-down

Inhibitory avoidance behavior is also studied, in a one-trial learning, step-down type situation (Proc Nat Acad Sci USA (2001) 98: 12251-12254). On the learning session, mice are gently placed on a 3.0-cm-high, 5.0-cm-wide platform (CS) at the left of a 20x20x15 cm black acrylic training apparatus, whose floor is a series of parallel 0.2-cm-caliber stainless-steel bars spaced 1.0 cm apart. Latency to step-down onto the grid with all four paws is measured and the animals receive a footshock (US) (0.8 mA, 50 Hz, 1 s). The drugs are administered immediately after training. Venlafaxine is administered in doses ranging from 0.3 to 10 mg/kg. Memantine is administered in doses ranging from 3 to 100 mg/kg. Forty-eight hours after administration of the drugs the retention test is performed. On the test session the footshock (US) is omitted. If a mouse fails to step-down within 300 s (ceiling score), the retention test is terminated and the mouse is assigned a score of 300 s. The step-down latency of the testing session is used as a measure of retention of the learned response.

### Drugs

The drugs used in these experiments were memantine and venlafaxine. Both drugs were dissolved in saline and were given intraperitoneally (10 ml/kg). The corresponding control groups received the same volume of saline. The doses of memantine and venlafaxine were calculated as the free base.

### Statistical Analysis

Behavioral data are expressed as median latencies to step-through or step-down during the retention test, and are analyzed, when appropriate, with the nonparametric analysis of variance of Kruskal-Wallis, and the differences between groups are estimated by individual Mann-Whitney U-tests (two tailed) (Siegel, 1956). In cases P values less than 0.05 are considered significant.

### EXAMPLE 5

An open-label, active-controlled comparative study is conducted in depressed patients with Alzheimer's disease. The objective of this study is to compare the efficacy of the osmotic device, containing a combination of venlafaxine and memantine, versus the administration of venlafaxine and memantine as two different products in immediate release formulations. Another objective is to establish the antidepressant and the cognitive effect of these products in patients diagnosed with Alzheimer's disease. This open-label, parallel-arm design study includes Alzheimer patients meeting DSM-TR IV criteria for major or minor depression who are randomized to receive either the one of the following treatments: 1) venlafaxine (controlled release) + memantine (immediate release); 2) venlafaxine (immediate release); 3) memantine (immediate release); and 4) venlafaxine (immediate release) and memantine (immediate release). A dose escalation lead-in period followed by a final maintenance-dose period is carried out for both, venlafaxine (37.5 to 300 mg/ daily) and memantine (10 to 40 mg/daily) during a minimum length of 8 to 10 weeks, according to each patient response to therapy.

Patients enrolled in this trial have a diagnosis of "probable" Alzheimer disease as defined by the National Institute of Neurological and Communicative disorders and Stroke and the Alzheimer's Disease and Related Disorders Association (NINCDS-ADRDA). The severity of dementia is assessed through the Minimental Status examination, which a range of 10 to 26 which fits the category of mild-to-moderate dementia; and also meet the DSM-TR IV criteria for major or minor depression. Elderly patients of both genders are enrolled.

All patients fortnightly receive evaluations consisting of the a) Alzheimer's Disease Assessment Scale-Cognitive (ADAS-Cog), b) the Clinician interview based Impression of change-Plus (CIBIC-Plus), c) the Alzheimer's Disease Cooperative Study-Activities of Daily living (ADCS-ADL), d) the Cornell Scale for Depression in Dementia, e) the Hamilton Depression Scale (HAM-D) and f) the Clinical Global Impression (CGI) as primary efficacy measures, and the g) Mini-Mental State Exam, h) the Hamilton Rating Scale for Anxiety, and i) the Functional Independence Measure as secondary efficacy measures. Standard safety evaluations and adverse event monitoring are carried out.

Improvement in the either one of the following scales (a, b, c, g & i) indicates a superior effect of the combined administration of venlafaxine in an osmotic device and memantine in an immediate release formulation over the administration of each agent alone in immediate release form or over the combined administration of venlafaxine and memantine in immediate release form, in terms of cognitive function.

Improvement in the either one of the following scales (d, e, f & h) indicates a superior effect of the combined administration of venlafaxine in an osmotic device and memantine in an immediate release formulation over the administration of each agent alone in immediate release form or over the combined administration of venlafaxine and memantine in immediate release form, in terms of antidepressant activity.

The above is a detailed description of particular embodiments of the invention. It is recognized that departures from the disclosed embodiments may be made within the scope of the invention and that obvious modifications will occur to a person skilled in the art. Those of skill in the art should, in light of the present disclosure, appreciate that many changes can be made in the specific embodiments which are disclosed herein and still obtain a like or similar result without departing from the spirit and scope of the invention. All of the embodiments disclosed and claimed herein can be made and executed without undue experimentation in light of the present disclosure.

## Claims

1. An osmotic device adapted for the administration of an anti-Alzheimer therapeutic composition, the osmotic device comprising:
a. a controlled release core comprising a therapeutically effective or sub-therapeutically effective amount of venlafaxine and at least one osmotic agent or osmopolymer;
b. a membrane surrounding the core and having one or more passageways there through; and
c. a rapid release drug-containing coat external to the membrane and comprising a therapeutically effective or sub-therapeutically effective amount of memantine;
d. wherein the external coat provides a rapid release of memantine, and at least 75% of the memantine is released within 1 hour after exposure of the osmotic device to an aqueous solution; and
e. the osmotic device provides a dispensable anti-Alzheimer therapeutic composition for administration of venlafaxine in a rate-controlled metered dose per unit time and memantine in a rapid release form.

2. The osmotic device of claim 1, wherein release of the memantine and venlafaxine occurs in a sequential manner.

3. The osmotic device of claim 1, wherein release of the memantine and venlafaxine occurs in an overlapping manner.

4. The osmotic device of claim 1, wherein the membrane is a semipermeable membrane.

5. The osmotic device of claim 1, wherein venlafaxine is provided in a therapeutically effective amount and memantine is provided in a sub-therapeutically effective amount.

6. The osmotic device of claim 1, wherein venlafaxine is provided in a sub-therapeutically effective amount and memantine is provided in a therapeutically effective amount.

7. The osmotic device of claim 1, wherein venlafaxine is provided in a therapeutically effective amount and memantine is provided in a therapeutically effective amount.

8. The osmotic device of claim 1, wherein venlafaxine is provided in a sub-therapeutically effective amount and memantine is provided in a sub-therapeutically effective amount.

9. The osmotic device of claim 1, wherein initial release of venlafaxine is delayed.

10. The osmotic device of claim 1, wherein venlafaxine is released according to the following release profile:
| Time (h) | Maximum percent released | Minimum percent released |
|---|---|---|
| 0 | 0 | 0 |
| 1 | 13 | 3 |
| 3 | 60 | 11 |
| 9 | 95 | 55 |
| 15 | 97 | 77 |
| 23 | 100 | 85 |

11. The osmotic device of claim 1, wherein venlafaxine and memantine are present in amounts sufficient to treat one or more symptoms associated with Alzheimer's disease and the osmotic device provides an at least additive clinical benefit when administered to a subject in need of such treatment.

12. A method of ameliorating one or more symptoms associated with Alzheimer's disease in a subject, the method comprising the steps of:
a. administering to the subject venlafaxine in controlled, extended or sustained release form; and
b. administering to the subject memantine in immediate or rapid release form.

13. The method of claim 12, wherein memantine and venlafaxine are administered in a sequential manner.

14. The method of claim 12, wherein memantine and venlafaxine are administered in an overlapping manner.

15. The method of claim 12, wherein memantine and venlafaxine are administered simultaneously.

16. The method of claim 12, wherein venlafaxine is administered in a therapeutically effective amount and memantine is administered in a sub-therapeutically effective amount.

17. The method of claim 12, wherein venlafaxine is administered in a sub-therapeutically effective amount and memantine is administered in a therapeutically effective amount.

18. The method of claim 12, wherein venlafaxine is administered in a therapeutically effective amount and memantine is administered in a therapeutically effective amount.

19. The method of claim 12, wherein venlafaxine is administered in a sub-therapeutically effective amount and memantine is administered in a sub-therapeutically effective amount.

20. The method of claim 12, wherein venlafaxine is administered in a controlled, extended, prolonged or sustained release form.

21. The method of claim 20, wherein venlafaxine is administered according to the following release profile:
| Time (h) | Maximum percent released | Minimum percent released |
|---|---|---|
| 0 | 0 | 0 |
| 1 | 13 | 3 |
| 3 | 60 | 11 |
| 9 | 95 | 55 |
| 15 | 97 | 77 |
| 23 | 100 | 85 |

22. The method of claim 12, wherein venlafaxine is administered according to a sigmoidal, zero order, pseudo-zero order, first order, or pseudo-first order release profile.

23. The method of claim 12, wherein venlafaxine and memantine are administered from the same dosage form.

24. The method of claim 12, wherein venlafaxine and memantine are administered from separate dosage forms.

25. The method of claim 24, wherein the separate dosage forms are provided in a kit.

26. The method of claim 12, wherein venlafaxine and memantine are administered in amounts sufficient to treat one or more symptoms associated with Alzheimer's disease and the method provides an at least additive clinical benefit to a subject in need of such treatment.

27. An osmotic device adapted for the administration of an anti-Alzheimer therapeutic composition, the osmotic device comprising:
a. a controlled release core comprising a therapeutically effective or sub-therapeutically effective amount of venlafaxine and at least one osmotic agent or osmopolymer;
b. a membrane surrounding the core and having one or more passageways there through; and
c. a rapid release drug-containing coat external to the membrane and comprising a therapeutically effective or sub-therapeutically effective amount of memantine;
d. wherein the external coat provides a rapid release of memantine, and at least 75% of the memantine is released within 1 hour after exposure of the osmotic device to an aqueous solution; and
e. the osmotic device provides a dispensable anti-Parkinson therapeutic composition for administration of venlafaxine in a rate-controlled metered dose per unit time and memantine in a rapid release form.

28. The osmotic device of claim 27, wherein release of the memantine and venlafaxine occurs in a sequential manner.

29. The osmotic device of claim 27, wherein release of the memantine and venlafaxine occurs in an overlapping manner.

30. The osmotic device of claim 27, wherein the membrane is a semipermeable membrane.

31. The osmotic device of claim 27, wherein venlafaxine is provided in a therapeutically effective amount and memantine is provided in a sub-therapeutically effective amount.

32. The osmotic device of claim 27, wherein venlafaxine is provided in a sub-therapeutically effective amount and memantine is provided in a therapeutically effective amount.

33. The osmotic device of claim 27, wherein venlafaxine is provided in a therapeutically effective amount and memantine is provided in a therapeutically effective amount.

34. The osmotic device of claim 27, wherein venlafaxine is provided in a sub-therapeutically effective amount and memantine is provided in a sub-therapeutically effective amount.

35. The osmotic device of claim 27, wherein initial release of venlafaxine is delayed.

36. The osmotic device of claim 27, wherein venlafaxine is released according to the following release profile:
| Time (h) | Maximum percent released | Minimum percent released |
|---|---|---|
| 0 | 0 | 0 |
| 1 | 13 | 3 |
| 3 | 60 | 11 |
| 9 | 95 | 55 |
| 15 | 97 | 77 |
| 23 | 100 | 85 |

37. The osmotic device of claim 27, wherein venlafaxine and memantine are present in amounts sufficient to treat one or more symptoms associated with Parkinson's disease and the osmotic device provides an at least additive clinical benefit when administered to a subject in need of such treatment.

38. A method of ameliorating one or more symptoms associated with Parkinson's disease in a subject, the method comprising the steps of:
a. administering to the subject venlafaxine in controlled, extended or sustained release form; and
b. administering to the subject memantine in immediate or rapid release form.

39. The method of claim 38, wherein memantine and venlafaxine are administered in a sequential manner.

40. The method of claim 38, wherein memantine and venlafaxine are administered in an overlapping manner.

41. The method of claim 38, wherein memantine and venlafaxine are administered simultaneously.

42. The method of claim 38, wherein venlafaxine is administered in a therapeutically effective amount and memantine is administered in a sub-therapeutically effective amount.

43. The method of claim 38, wherein venlafaxine is administered in a sub-therapeutically effective amount and memantine is administered in a therapeutically effective amount.

44. The method of claim 38, wherein venlafaxine is administered in a therapeutically effective amount and memantine is administered in a therapeutically effective amount.

45. The method of claim 38, wherein venlafaxine is administered in a sub-therapeutically effective amount and memantine is administered in a sub-therapeutically effective amount.

46. The method of claim 38, wherein venlafaxine is administered in a controlled, extended, prolonged or sustained release form.

47. The method of claim 46, wherein venlafaxine is administered according to the following release profile:
| Time (h) | Maximum percent released | Minimum percent released |
|---|---|---|
| 0 | 0 | 0 |
| 1 | 13 | 3 |
| 3 | 60 | 11 |
| 9 | 95 | 55 |
| 15 | 97 | 77 |
| 23 | 100 | 85 |

48. The method of claim 38, wherein venlafaxine is administered according to a sigmoidal, zero order, pseudo-zero order, first order, or pseudo-first order release profile.

49. The method of claim 38, wherein venlafaxine and memantine are administered from the same dosage form.

50. The method of claim 38, wherein venlafaxine and memantine are administered from separate dosage forms.

51. The method of claim 50, wherein the separate dosage forms are provided in a kit.

52. The method of claim 38, wherein venlafaxine and memantine are administered in amounts sufficient to treat one or more symptoms associated with Parkinson's disease and the method provides an at least additive clinical benefit to a subject in need of such treatment.
